(19) Europäisches Patentamt
European Patent Office
Office européen des brevets

(11) Veröffentlichungsnummer: **0 182 190**
**A2**

(12) # EUROPÄISCHE PATENTANMELDUNG

(21) Anmeldenummer: **85114023.6**

(22) Anmeldetag: **05.11.85**

(51) Int. Cl.⁴: **C 07 D 239/54**

(30) Priorität: **16.11.84 DE 3441935**

(43) Veröffentlichungstag der Anmeldung: **28.05.86**
**Patentblatt 86/22**

(84) Benannte Vertragsstaaten: **CH DE FR GB LI**

(71) Anmelder: **BAYER AG, Konzernverwaltung RP**
**Patentabteilung, D-5090 Leverkusen 1 Bayerwerk (DE)**

(72) Erfinder: **Herd, Karl Josef, Dr., Am Gartenfeld 66,**
**D-5068 Odenthal (DE)**
Erfinder: **Schündehütte, Karl Heinz, Prof. Dr., Klief 75,**
**D-5090 Leverkusen 3 (DE)**

(54) **Verfahren zur Herstellung von 2,4-Dihydroxypyrimidinen.**

(57) 2,4-Dihydroxypyrimidine der Formel

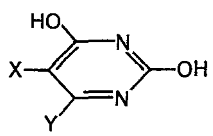

mit den in der Beschreibung angegebenen Substituentenbedeutungen erhält man durch Umsetzung von Pyrimidinen der Formel

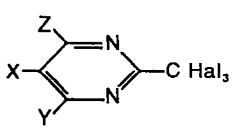

bzw.

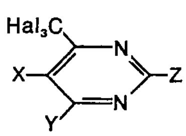

mit den in der Beschreibung angegebenen Substituentenbedeutungen mit wäßrigen Alkalien bei erhöhter Temperatur. Aus den Dihydroxypyrimidinen erhält man durch Umsetzung mit Hydroxy/Chlor-Austauschreagentien 2,4,5,6-Tetrachlorpyrimidin.

EP 0 182 190 A2

BAYER AKTIENGESELLSCHAFT 5090 Leverkusen, Bayerwerk
Konzernverwaltung RP
Patentabteilung My/ABc

# Verfahren zur Herstellung von 2,4-Dihydroxypyrimidinen

Gegenstand der vorliegenden Erfindung ist ein Verfahren zur Herstellung von 2,4-Dihydroxypyrimidinen (1H, 3H-2,4-Pyrimidindionen), die in einer der möglichen tautomeren Formen der Formel

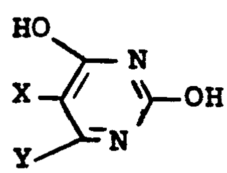

(1)

entsprechen,

worin X = H, Cl, Br oder gegebenenfalls substituiertes $C_1$-$C_4$-Alkyl und

Y = X oder OH.

Vorzugsweise steht X für Cl oder Br und Y für Cl.

Das neue Herstellungsverfahren für die Verbindungen (1) besteht darin, daß man Trihalogenmethylverbindungen der Formel (2) bzw. (3),

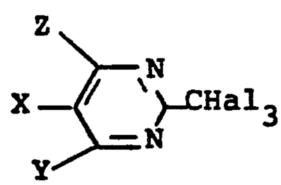

(2)

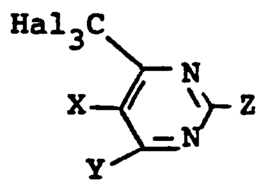

(3)

worin Hal = Cl oder Br,

Z = Hal oder Hydroxy und

X und Y die oben genannte Bedeutung haben,

in wäßrig-alkalischen Medium gegebenenfalls unter Druck erhitzt. Gegebenenfalls können bei dieser Umsetzung wassermischbare Lösungsmittel wie Sulfolan, Acetonitril, Dimethylsufoxid oder Dimethylformamid mitverwendet werden.

Vorzugsweise führt man die Umsetzung mit etwa 10-50 %ige Natron- oder Kalilauge, insbesondere etwa mit 10-30 %iger Natronlauge bei Temperaturen zwischen 60-110°C durch. Unter diesen Reaktionsbedingungen wird das bei der Reaktion freiwerdende Haloform direkt hydrolysiert.

Die Verbindungen (2) und (3) sind aus der Literatur bekannt bzw. lassen sich nach literaturbekannten Verfahren herstellen.

Die 2,4-Dihydroxypyrimidine (1) sind wertvolle Zwischenprodukte zur Herstellung von chlor- oder fluorhaltigen

Pyrimidinen, wie 2,4,5,6-Tetrachlorpyrimidin oder 5-Chlor-2,4,6-trifluorpyrimidin, die als Reaktivkomponenten in der Farbstoffchemie von Bedeutung sind, und die man durch Reaktion von (1) mit beispielsweise Phosphoroxychlorid, Phosphorpentachlorid, Phosphortrichlorid, Thionylchlorid, Phosgen oder ähnlichen Hydroxy/Chlor-Austauschreagentien erhält.

Das neue Verfahren findet besonders zur Herstellung folgender Dihydroxypyrimidine der Formel (1) Verwendung:
5,6-Dichlor-2,4-dihydroxypyrimidin,
6-Chlor-2,4-dihydroxypyrimidin,
5-Brom-6-chlor-2,4-dihydroxypyrimidin,
6-Chlor-5-methyl-2,4-dihydroxypyrimidin,
5,6-Dibrom-2,4-dihydroxypyrimidin.

Einzelheiten zur Darstellung sind in den Beispielen aufgeführt.

Beim Arbeiten in hohen Konzentrationen, insbesondere hohen Alkalikonzentrationen können sich zunächst Dimere und/oder Trimere bilden, die mit fortschreitender Reaktionsdauer oder Verdünnen der Reaktionsmischung zu den monomeren Verbindungen hydrolysieren.

Für das Herstellungsverfahren sind vorzugsweise folgende Trihalogenmethylpyrimidine (2) bzw. (3) als Ausgangsverbindungen von Interesse:

4,5,6-Trichlor-2-trichlormethylpyrimidin,
2,5,6-Trichlor-4-trichlormethylpyrimidin,
4,6-Dichlor-2-trichlormethylpyrimidin,
2,6-Dichlor-4-trichlormethylpyrimidin,
5,6-Dichlor-4-hydroxy-2-trichlormethylpyrimidin,
5-Brom-4,6-dichlor-2-trichlormethylpyrimidin,
6-Chlor-4-hydroxy-2-trichlormethylpyrimidin,
6-Chlor-2-hydroxy-4-trichlormethylpyrimidin,
5,6-Dichlor-2-hydroxy-4-trichlormethylpyrimidin,
2,6-Dichlor-5-methyl-4-trichlormethylpyrimidin,
5-Brom-6-chlor-4-hydroxy-2-trichlormethylpyrimidin,
4,5,6-Tribrom-2-tribrommethylpyrimidin.

## Beispiel 1

**5,6-Dichlor-2,4-dihydroxypyrimidin aus 4,5,6-Trichlor-2-trichlormethylpyrimidin**

A) 242 g geschmolzenes 4,5,6-Trichlor-2-trichlormethylpyrimidin werden innerhalb von 1,5 Stunden über einen beheizten Tropftrichter zu einer 75-80°C warmen Vorlage aus 1200 ml Wasser, 600 ml 50 %iger Natronlauge und 15 ml einer Emulgatorlösung zudosiert. Dabei ist infolge exothermer Reaktionswärme ein Temperaturanstieg auf 95°C zu beobachten. Es resultiert eine klare bernsteinfarbene Lösung. Nach einer weiteren Stunde bei 90-95°C wird abgekühlt, und die Reaktionslösung mit Salzsäure auf pH 6 eingestellt. Das auskristallisierte Produkt wird abgesaugt, mit ca. 70 ml Wasser nachgewaschen und getrocknet. Man erhält 119 g (82 % d.Th.) einer sandfarbenen kristallinen Substanz vom Schmp. 302-303°C (Zers.). Die Substanz erweist sich mit 5,6-Dichlor-2,4-dihydroxypyrimidin, das durch Hydrolyse von 2,4,5,6-Tetrachlorpyrimidin nach DE-OS 215 3511 erhalten wurde, im IR- und Schmp.-Vergleich als identisch. Im Filtrat ist ein geringer Anteil an 5,6-Dichlor-4-hydroxy-2-pyrimidincarbonsäure nachweisbar.

B) 500 g einer ca. 50 %igen Lösung von 4,5,6-Dichlor-2-trichlormethylpyrimidin in Sulfolan werden zu 1000 ml 40 %ige Natronlauge, die auf 70°C erwärmt wurde, langsam zugetropft. Man rührt eine weitere

Stunde bei 90°C. Dabei ist eine Abscheidung von oligomeren Zwischenprodukten zu beobachten. Man versetzt mit 2 l Wasser und erwärmt die Reaktionsmischung solange unter Rückflußkühlung, bis eine relativ klare Lösung resultiert. Nach Abkühlen, Neutralisieren, Absaugen und Trocknen analog Variante A) werden 115 g 5,6-Dichlor-2,4-dihydroxypyrimidin isoliert.

Beispiel 2

5,6-Dichlor-2,4-dihydroxypyrimidin aus 2,5,6-Trichlor-4-trichlormethylpyrimidin

120 g 2,5,6-Trichlor-4-trichlormethylpyrimidin werden bei 70°C in eine Lösung aus 1000 ml Wasser, 500 ml 40 %-iger Kalilauge und 10 ml Emulgatorlösung portionsweise eingetragen. Nach einer weiteren Stunde bei 65-70°C wird die warme Reaktionslösung geklärt, abgekühlt und auf pH 4 angesäuert. Es werden nach Isolierung und Trocknung 57 g (79 % d.Th.) 5,6-Dichlor-2,4-hydroxypyrimidin erhalten.

Beispiel 3

6-Chlor-2,4-dihydroxypyrimidin aus 4,6-Dichlor-2-trichlormethylpyrimidin

10 g 4,6-Dichlor-2-trichlormethylpyrimidin werden in 50 ml konz. Natronlauge und 100 ml Wasser unter Emulgatorzusatz auf 90°C erwärmt. Vorübergehend tritt ein

Zweiphasensystem, später eine Ausfällung und zuletzt eine trübe Lösung auf. Nach weiteren 30 Minuten Rühren bei 90°C wird auf 50°C abgekühlt, geklärt und die Lösung mit Salzsäure neutralisiert. Anschließend wird die Lösung bis zur beginnenden Kristallisation aufkonzentriert und im Eisbad abgekühlt oder aber mit 20 g Kochsalz ausgesalzen. Das kristalline Produkt wird abgesaugt, mit wenig Eiswasser gewaschen und getrocknet. Es werden 3,7 g (68 % d.Th.) 6-Chlor-2,4-dihydroxypyrimidin vom Schmp. 300°C (Zers.) (vgl. J. Chem. Soc. 1960, 4771) erhalten.

Beispiel 4

5,6-Dichlor-2,4-dihydroxypyrimidin aus 5,6-Dichlor-4-hydroxy-2-trichlormethylpyrimidin

282,5 g bei 120°C geschmolzenes 5,6-Dichlor-4-hydroxy-2-trichlormethylpyrimidin werden langsam in eine 100-108°C warme Vorlage aus 600 ml 40 %iger Natronlauge eingetragen. Durch die freiwerdende Reaktionswärme wird die Reaktionslösung konstant auf Rückflußtemperatur gehalten. Die Dauer des Eintragen beträgt 2 Stunden. Danach ist auch die Reaktion beendet. Man kühlt auf 25°C ab und stellt mit 100 ml Salzsäure auf pH 5. Das ausgefallene blaßgelbe Produkt wird abgesaugt und getrocknet. Die Ausbeute an 5,6-Dichlor-2,4-dihydroxypyrimidin beträgt 163 g (90 % d.Th.).

Beispiel 5

2,4,5,6-Tetrachlorpyrimidin (TCP)

163 g 5,6-Dichlor-2,4-dihydroxypyrimidin werden in eine Mischung aus 230 g Phosphoroxychlorid, 25 g Phosphortrichlorid und 10 g Tri-n-Butylamin eingetragen und 6 Stunden unter Rückfluß erwärmt, bis die Temperatur in der Dampfphase auf 110°C ansteigt. Innerhalb von 10 Stunden leitet man langsam und gleichzeitig 300 g Phosphortrichlorid und 125 g Chlor ein. Abschließend versetzt man nochmals mit 19 g Chlor und erwärmt eine weitere Stunde, wobei die Temperatur 117°C erreicht. Aus der klaren Lösung wird zunächst Phosphoroxychlorid abdestilliert, anschließend das Tetrachlorpyrimidin im Vakuum fraktioniert. Die Ausbeute beträgt 183 g.

## Patentansprüche

1. Verfahren zur Herstellung von 2,4-Dihydroxypyrimidinen, die in einer der möglichen tautomeren Formen der Formel

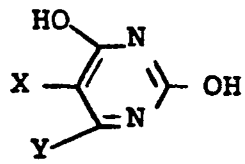

entsprechen, worin

X = H, Cl, Br, gegebenenfalls substituiertes $C_1$-$C_4$-Alkyl,

Y = X oder OH,

dadurch gekennzeichnet, daß man Pyrimidine der Formel

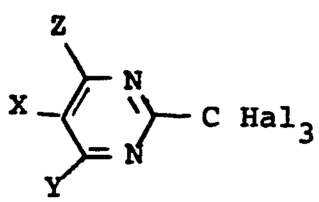

bzw.

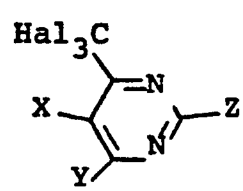

worin

Hal = Cl oder Br,
Z = Hal oder OH

in wäßrig-alkalischen Medium erhitzt.

2. Verfahren nach Anspruch 1, dadurch gekennzeichnet, daß man die Reaktion bei etwa 60-110°C mit etwa 10-50 %iger NaOH oder KOH durchführt.

3. Verfahren zur Herstellung von 2,4,5,6-Tetrachlorpyrimidin, dadurch gekennzeichnet, daß man 5,6-Dichlor-2,4-dihydroxypyrimidin mit Hydroxy/Chlor-Austauschreagentien umsetzt.

4. Verfahren nach Anspruch 3, dadurch gekennzeichnet, daß man als Hydroxy/Chlor-Austauschreagenz Phosphorpentachlorid, Phosphortrichlorid, Phosphoroxychlorid, Thionylchlorid oder Phosgen verwendet.

5. Verfahren zur Herstellung von 2,4,5,6-Tetrachlorpyrimidin, dadurch gekennzeichnet, daß man gemäß Ansprüchen 1-2 hergestelltes 5,6-Dichlor-2,4-dihydroxypyrimidin gemäß Ansprüchen 3 und 4 weiter umsetzt.